# EUROPEAN PATENT APPLICATION

(11) **EP 1 437 090 A1**
(43) Date of publication of application: **14.07.2004**
(21) Application number: 02799489.6
(22) Date of filing: 19.09.2002
(51) Int. Cl.: A61B 5/053, A61B 5/04

(54) **AUTOMATIC, CONTINUOUS MEASURING DEVICE FOR A VENTRICLE VOLUME**

(30) Priority: 20.09.2001 JP 2001286997
(71) Applicant: Japan as represented by president of National Cardiovascular Center, Suita-shi, Osaka 565-8565 (JP); Japan Aerospace Exploration Agency, Tsukuba-shi, Ibaraki 305-8505 (JP); The Japan Space Forum, Minato-ku, Tokyo 105-0013 (JP)
(72) Inventor: SUNAGAWA, Kenji, Ibaraki-shi, Osaka 567-0048 (JP); UEMURA, Kazunori, Toyonaka-shi, Osaka 560-0005 (JP)
(74) Representative: Wilson, Peter David
(86) International application number: PCT/JP2002/009655
(87) International publication number: WO 2003/026504

(57) **Abstract**

**Summary**

The purpose of the present invention is to provide an automatic, continuous measuring device for a ventricle volume that is able to measure blood conductivity and parallel conductance, which are regarded as necessary for the calculation of ventricular volume from the conductance measured, using a conductance catheter inserted into the ventricle without special procedures such as blood sampling and saline infusion.

An automatic, continuous measuring device for a ventricle volume with the following characteristics provides the means to solve the above-mentioned purpose: it is furnished with a multifunction catheter (2) to be introduced into the ventricle and a power supply unit (5) to supply the necessary current to the above-mentioned multifunction catheter (2); and that the above-mentioned multifunction catheter (2) is composed of two parts - a conductance catheter (3) containing multiple segments (31s₁ to 31s₅) interposed with electrodes (32d₁ to 32d₆) at fixed intervals with an arbitrary high-frequency weak current passing between the two end electrodes (32d₁ and 32d₆), and a electrode (4) for measuring blood conductivity placed in close proximity to one of the above-mentioned multiple electrodes (32d₁ to 32d₆).

## Description

### Technical Field

This invention concerns an automatic, continuous measuring device for a ventricle volume, and in detail, concerns an automatic, continuous measuring device for a ventricle volume using a conductance catheter. The purpose is to provide an automatic, continuous measuring device for a ventricle volume by which blood conductivity and parallel conductance, which are regarded as essential for calculating ventricular volume from the conductance measured from a conductance catheter, can be measured by the conductance catheter inserted into the ventricle, and does not require special procedures such as blood sampling and hypertonic saline infusion.

### Background technology

Recording an electrocardiogram is one of the conventional methods for investigating cardiac function. Although this method is relatively simple and widely used, electrocardiographic signals are indirect signals expressed outside the human body. It is thus difficult to understand the precise condition of the heart from the electrographic signals, and interpretation of the signals requires skill. In addition, some symptoms do not manifest in the electrocardiographic signals and there is a risk of overlooking cardiac abnormalities.

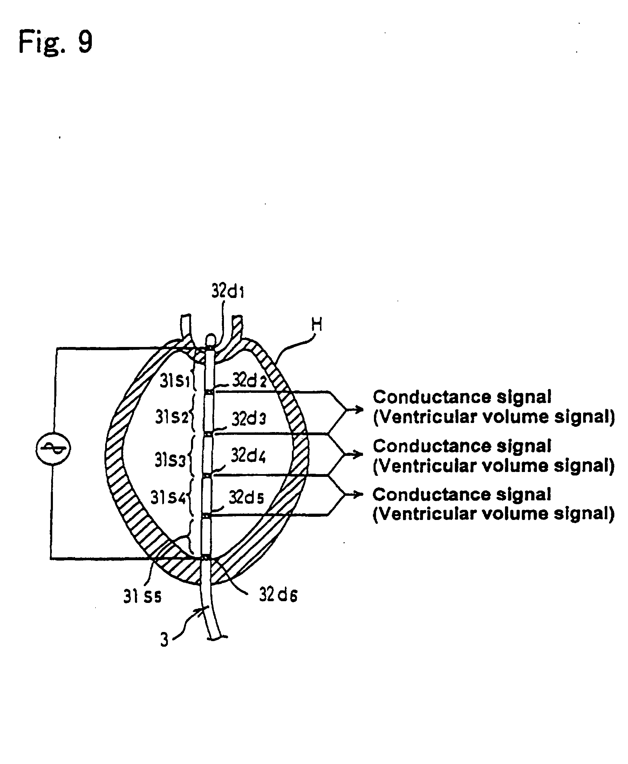

On the other hand, ventricular volume measurement is an important factor for the quantitative evaluation of the cardiac function. A known method of measuring ventricular volume is to use the one-dimensional or two-dimensional measurements obtained from echocardiography or magnetic resonance imaging (MRI) and then apply a revolution ellipsoidal model. However, these methods require a very long measuring time and are not direct measurements of the ventricular volume. Moreover, a lack of reliability is a major problem. On the other hand, the conductance catheter method is a known method that directly measures the ventricular volume. As shown in Figure 9, this method requires a conductance catheter (3) composed of several (5 in the example shown in the figure) segments (31s₁ to 31s₅) along the longitudinal direction. This catheter is inserted into the apical portion of the heart (H) and directed toward the aortic valve. A weak current with specific high frequencies (such as 20 KHz and 30 µA) flows constantly between the electrodes (32d₁ and 32d₆) at the two ends of this catheter (3). The voltage between the electrodes (32d₂ and 32d₃) (32d₃ and 32d₄, or 32d₄ and 32d₅) at two ends of each of the inner segments (31s₂) (31s₃ or 31s₄) is measured. When the above mentioned high-frequency weak current passes through the conductance catheter (3), a three-dimensional electric field is formed in the ventricle with the blood in the ventricle acting as the medium. This change in electric field, or change in conductance (the reciprocal of impedance) is measured as voltage change between the electrodes (32d₂ and 32d₃) (32d₃ and 32d₄, or 32d₄ and 32d₅) at the two ends of segment 31s₂ (31s₃ or 31 s₄).

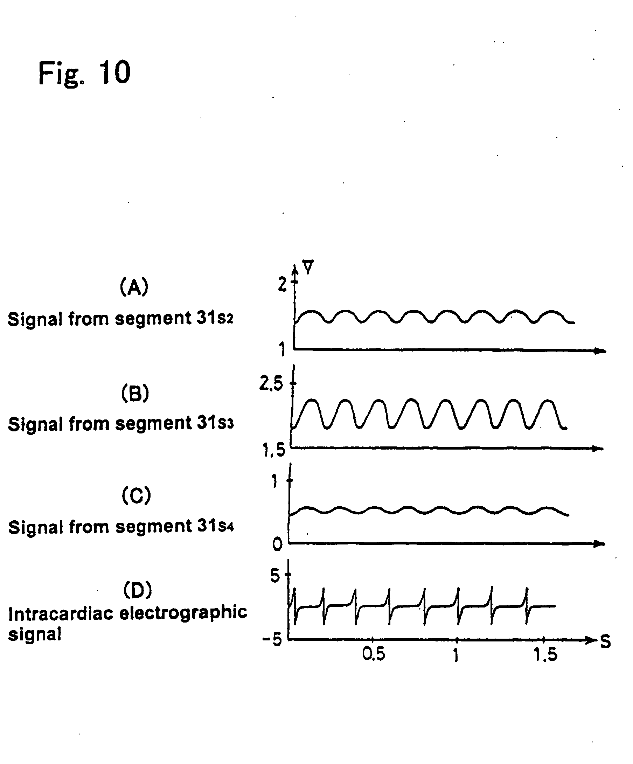

Figure 10 shows the wave diagrams of the signals obtained from the above conductance catheter. (A) to (C) show the voltage changes between electrodes at the two end of the each of the inner segments. (D) shows the intracardiac electrocardiographic signals.

Since a fixed relation exists between the conductance at each segment and the ventricular volume, the ventricular volume can be calculated by measuring the conductance at each segment.

The conductance catheter used in the conductance catheter method is known to be disclosed in, for example, Japan patent publication No. 269136/1993 or Japan patent publication No. 1372090/1998.

However, although the method of using conductance catheter to measure ventricular volume is superior in temporal resolution compared to other methods, several problems exist, as shown below.

To calculate the ventricular volume from the conductance measured by the conductance catheter, various calibrations are regarded necessary, such as blood conductivity measured by blood sampling and parallel conductance measured by hypertonic saline infusion. This means that the operator who measures the conductance from a conductance catheter implanted in an experimental animal has to be at the side of the experimental animal to sample blood and conduct other essential procedures, and the ventricular volume cannot be measured automatically by the conventional methods. For this reason, when the experimental animal and the operator are geographically separated from each other, such as in the case of measuring ventricular volume in space environments such as the space ship and space station, the operator has no choice but to accompany the animal. In addition, when the conductance is implanted long-term for conductance measurements, these test procedures become cumbersome.

This invention is a device for continuous ventricular volume measurements by automatic procedures using a conductance catheter. The conductance catheter inserted into the ventricle allows the measurement of blood conductivity and parallel conductance, which are regarded as essential for calculating ventricular volume from the conductance measured from the conductance catheter, thereby obviating the need for special procedures such as blood sampling and hypertonic saline infusion. Therefore, it is an automatic, continuous measuring device for a ventricle volume that allows automatic and moreover continuous measurement of ventricular volume even though the operator is not at the side of the animal implanted with a conductance catheter.

### Disclosure of the invention

The invention described in claim 1 concerns an automatic, continuous measuring device for a ventricle volume with the following features: that it is furnished with a multifunction catheter to be inserted into the ventricle and a power supply unit that supplies the necessary current to the above-mentioned multifunction catheter; and that the above-mentioned multifunction catheter is composed of two parts - a conductance catheter divided into multiple segments with electrodes interposed at fixed intervals such that an arbitrary high-frequency weak current is passed between the two end electrodes among the above-mentioned electrodes while the voltage differences between each of the segments except the two end segments are measured, and an electrode for measuring blood conductivity placed in one of the above-mentioned multiple segments.

The invention described in claim 2 concerns an automatic, continuous measuring device for a ventricle volume with the following features: that it is furnished with a multifunction catheter to be inserted into the ventricle and a power supply unit that supplies the necessary current to the above-mentioned multifunction catheter; that the above-mentioned multifunction catheter is composed of two parts - a conductance catheter divided into at least three segments with at least four electrodes fitted at fixed intervals such that an arbitrary high-frequency weak current is passed between the two end electrodes among the above-mentioned electrodes while the voltage differences between each of the segments except the two end segments are measured, and 4 electrodes for measuring blood conductivity placed in one of the above-mentioned segments; and that among the above-mentioned four electrodes, the two end electrodes are designated current input electrodes and the pair of inner electrodes is designated voltage measurement electrodes.

The invention described in claim 3 concerns an automatic, continuous measuring device for a ventricle volume described in claim 1 or claim 2 with the following feature: that the above-mentioned power supply unit is configurated so as to be able to supply a weak current at two frequencies between the two electrodes at the two ends of the above-mentioned multifunction catheter.

The invention described in claim 4 concerns an automatic, continuous measuring device for a ventricle volume described in claim 1 or claim 2 with the following feature: that the above-mentioned power supply unit is configurated so as to be able to supply a weak current at three or more frequencies between the two electrodes at the two ends of the above-mentioned multifunction catheter; and that individual ventricular conductances are measured with three or more arbitrary frequencies supplied to the electrodes at the two ends of the above-mentioned multifunction catheter, and the two frequencies that provide the greatest difference in conductance are used to determine parallel conductance.

### Brief description of the drawings

Figure 1 is an enlarged drawing of a part of the multifunction catheter.
Figure 2 is a block diagram showing the outline of the automatic, continuous measuring device for a ventricle volume concerning the first embodiment of this invention.
Figure 3 is a block diagram showing the outline of the automatic, continuous measuring device for a ventricle volume concerning the second embodiment of this invention.
Figure 4 is a block diagram of an enlarged and extracted part from the automatic, continuous measuring device for a ventricle volume shown in Figure 3.
Figure 5 is a graph showing the results of Test 1, and the graph shows the relation between the effective conducting volume and the inter-electrode distance, when a constant current is flowed between the two electrodes on the catheter.
Figure 6 is a graph showing the results of Test 2, and the graph shows the correlation between blood conductivity (σc) measured in a cuvette and the blood conductivity (σ v) measured using the catheter.
Figure 7 is a graph showing the correlation between the parallel conductance (GpDF) calculated from the mean difference in conductance signal (ΔG 2KHz-20KHz) and the parallel conductance (GpSAL) measured by the hypertonic saline infusion method at 20 KHz.
Figure 8 is a graph showing the correlation between the diastolic and systolic left ventricular volume (Vc) calculated by the conventional method and the diastolic and systolic left ventricular volume (Ve) calculated form the invented device.
Figure 9 is a diagram showing the in use state of the conductance catheter.
Figure 10 shows the waveforms of the signals obtained from the conductance catheter shown in Figure 9.

### Best mode for carrying out the invention

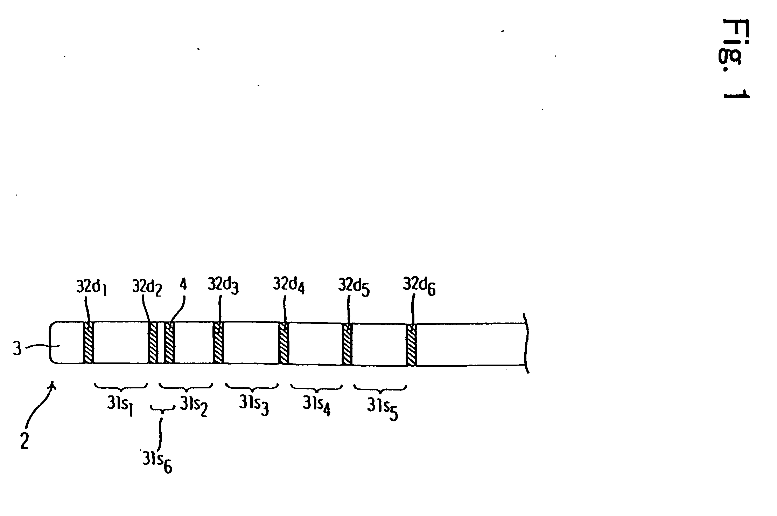

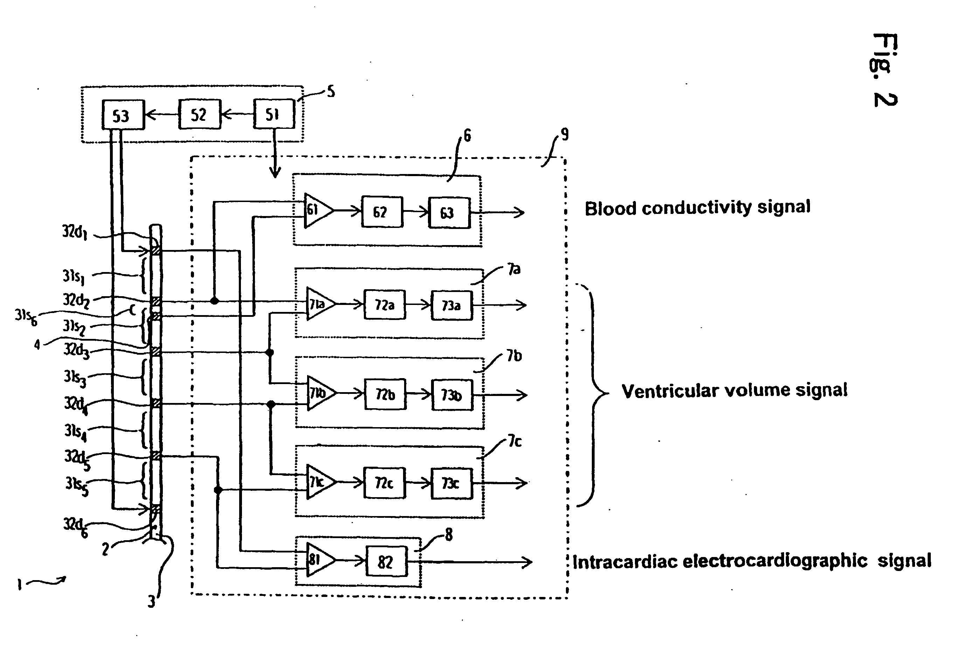

The automatic, continuous measuring device for a ventricle volume concerning the first embodiment of this invention will be explained below with reference to the drawings. Figure 1 is an enlarged drawing of a part of the multifunction catheter. Figure 2 is a block diagram showing the outline of the automatic, continuous measuring device for a ventricle volume concerning the first embodiment of this invention.

The automatic, continuous measuring device for a ventricle volume (1) concerning the first embodiment of this invention is composed of a multifunction catheter (2), a power supply unit (5) and the main unit (9).

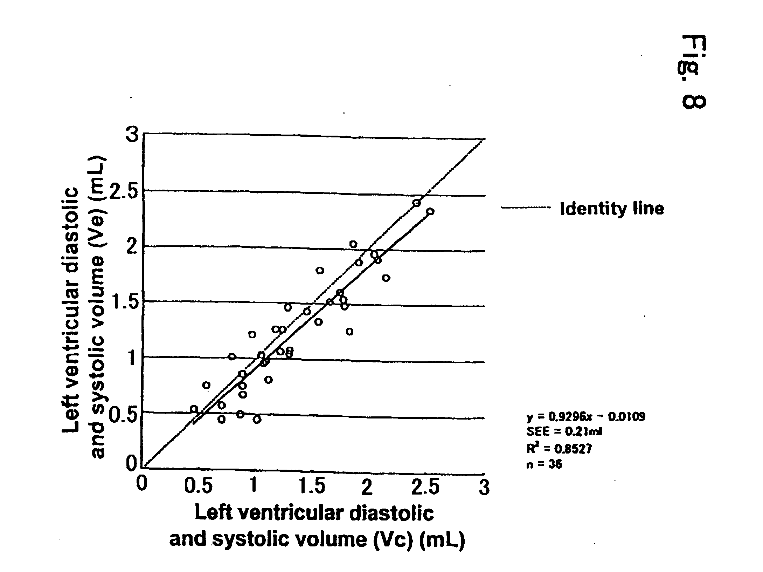

The multifunction catheter (2) is to be inserted into the ventricle, and consists of a conductance catheter (3) and an electrode for measuring blood conductivity (4), as shown in an enlarged diagram in Figure 1. The conductance electrode (3) is similar to the conventional conductance catheter as explained in Figure 8, and is composed of multiple (five in the example shown in the figure) segments (31s₁ to 31s₅) interposed with electrodes (32d₁ and 32d₆) at fixed intervals. The multiple (five in the example shown in the figure) segments (31s₁ to 31s₅) each with electrodes at the two ends are arranged together along the longitudinal direction. Then an arbitrary high-frequency weak current, such as 20 KHz and 3-5 µA supplied from a power supply unit to be described later is passed between the two electrodes (32d₁ and 32d₆) located at the outer side of the two end segments (31s₁ and 31s₅, respectively). The voltage changes for the remaining segments (31s₂ to 31s₄) (thereinafter referred to as conductance measuring segments) except the two end segments (31s₁ and 31s₅) are measured.

The number of conductance measuring segments and the inter-electrode distance of the electrodes (32d₁ to 32d₆) are not restricted, and these can be set appropriately according to the size of the heart to be measured. In general, it is considered that at least one conductance measuring segment is required to measure voltage changes in the case of measuring the ventricular volume of the mouse heart, at least three conductance measuring segments to measure voltage changes in the case of measuring the ventricular volume of the rat heart, and at least five conductance measuring segments to measure voltage changes in the case of measuring the ventricular volume of the human heart. The electrodes can be placed at distances that allows placement of the above-mentioned number of conductance measuring segments. For example, in the case of measuring the ventricular volume of the rat heart, setting an inter-electrode distance of approximately 2 mm will allow adequate space for the above-mentioned number of conductance measuring segments.

The electrode for measuring blood conductivity (4) is placed at one of the above-mentioned multiple segments. In other words, it is placed close to one (32d₂ in the figure) of the above-mentioned multiple electrodes (32d₁ to 32d₆), and blood conductivity is measured by the two electrodes (4 and 32d₂) at the two ends of the segment (31 s₆) by the two-electrode measurement method. The reason for placing the electrode for measuring blood conductivity close to one (32d₂ in the figure) of the above-mentioned multiple electrodes (32d₁ to 32d₆) is that since the cardiac volume changes constantly as a result of heart beat, blood conductivity cannot be measured due to the pulse-induced volume changes if the electrode for measuring blood conductivity (4) is far apart from one other of the above-mentioned multiple electrodes (32d₁ to 32d₆). Therefore, the electrode for measuring blood conductivity and one of the above-mentioned multiple electrodes (32d₁ to 32d₆) are placed at a distance that is not affected by the ventricular volume change accompanying beating of the heart; in other words, at a close enough distance such that the current from one electrode is converged only within the ventricular blood.

As a practical example, in the case of a Japanese white rabbit in which the ventricular diameter dilates and contracts repeatedly in the range of approximately 6 to 12 mm, it is desirable to place the electrode for measuring blood conductivity at a distance of approximately 0.5 mm from one of the above-mentioned multiple electrodes.

The power supply unit (5) consists of a battery (51), a high frequency power circuit (52) and a frequency modulating circuit (53). The battery (51) supplies the necessary power to all parts of the signal detectors (7) and all parts of the automatic, continuous measuring device for a ventricle volume (1). In addition, the high frequency power circuit (52) supplies an arbitrary high-frequency weak current that flows between the two electrodes (32d₁ and 32d₆) at the two ends of the conductance catheter (3). Furthermore, the frequency modulating circuit (53) is installed so that the frequency of the weak current supplied to the conductance catheter (3) can be modulated arbitrarily.

Therefore, by measuring the intra-ventricular conductance using two different frequencies (for example, 20 KHz and 2 KHz); in other words, utilizing the difference in frequency characteristics of conductivity between blood and myocardium, it is possible to measure the parallel conductance derived from the myocardium and other sources. Therefore, parallel conductance, which is conventionally measured by hypertonic saline infusion and other methods, can be measured without special procedures.

In the measurement of parallel conductance using different frequencies, while the parallel conductance derived from the myocardium and other sources can be estimated by measuring ventricular conductance using two predetermined frequencies such as "20 KHz and 2 KHz", it is also possible to measure the ventricular conductance individually using three or more arbitrary frequencies (for example, 2 KHz, 20 KHz and 30 KHz) and then choose the two frequencies (for example, 2 KHz and 30 KHz) that give the greatest difference in conductance, and use them to determine parallel conductance. Using these methods, the parallel conductance, which is conventionally measured by hypertonic saline infusion and other methods, can be measured without special procedures and, moreover, with higher precision.

The signal detector (7) is the part that detects ventricular volume signals, intracardiac electrocardiographic signals and blood conductivity signals from the multifunction catheter (2). As shown in Figure 2, the signal input side consists of multiple (three in the figure) signal detectors (7a, 7b and 7c) (hereinafter referred to as 7 collectively) for detecting conductance signals that are the ventricular volume signals, one signal detector (8) for intracardiac electrocardiographic signals, and one signal detector (6) for blood conductivity signals. From these signal detectors (6, 7 and 8), the detected signals are output via four or more channels (five channels in the figure).

Among the above-mentioned signal detectors (6, 7 and 8), the detectors (7) for ventricular volume signals are installed corresponding to the three conductance measuring segments (31s₂, 32s₃, 32s₄) in the inner part of the conductance catheter (3). They are composed of differential amplifiers (71a, 71 b, 71c) to measure signals from the electrodes (32d₂ and 32d₃) (32d₃ and 32d₄, or 32d₄ and 32d₅) at the two ends of each of the inner conductance measuring segments (31 s₂) (31 s₃ or 31s₄), band pass filters (72a, 72b and 72c), and wave detectors (73a, 73b and 73c). The band pass filters (72a, 72b and 72c) eliminate contamination by other signals such as intracardiac electrocardiographic signals. The wave detectors (73a, 73b and 73c) cut off the high frequency components from the output of the band pass filters (72a, 72b and 72c) and extract the low frequency components.

The signal detector (8) for intracardiac electrocardiographic signals consists of a differential amplifier (81) connected to the two electrodes (32d₁ and 32d₅) at the two ends of the conductance catheter (3), and a band pass filter (82).

The signal detector (6) for blood conductivity signals consists of a differential amplifier (61) connected to the electrode for measuring blood conductivity (4) and a electrode (32d₂ in the figure) located close to the above electrode, and a band pass filter (62).

In addition, a pressure sensor (not shown in the figure) for measuring ventricular pressure may also be installed in the above-mentioned multifunction catheter (2). By installing a pressure sensor, the state of the heart can be monitored more precisely. The type of pressure sensor is not restricted, but use of a piezo-resistant element is desirable. The reason is that this type of sensor is superior with respect to response to mild pressure changes, and is able to accurately measure the mild changes of ventricle pressure compared to pressure sensors using piezoelectric element. For a pressure sensor utilizing piezo-resistant element, a bias alternating voltage is input from the power supply unit (5). The signals detected by the pressure sensor is sent to a signal detector (not shown in the figure) for ventricular pressure signals, which has a differential amplifier (not shown in the figure), and are then output from the signal detector as ventricular pressure signals.

The signals output from the above-mentioned signal detectors (6, 7 and 8) are sent to the computing and processing unit (not shown in the figure) and undergo specified processing.

On this occasion, these signals can be sent either by wired or wireless route. For example, if the signals are sent by wireless method, in the above-mentioned components, the signals output from various signal detectors (6, 7 and 8) are sent to a multiple converter. In this multiple converter, the signals from various channels are sampled in sequence, and converted to pulse position modulation (PPM wave) in the order of sampling, and the signals from multiple channels are subjected to time-division multiplexing. The pulse sequences of the modulated pulses including signals from multiple channels are superimposed on fixed carrier waves and transmitted by the transmission unit.

Next, the operations of the automatic, continuous measuring device for a ventricle volume concerning the first embodiment of this invention will be explained.

For measurement, the multifunction catheter (2) is inserted into the ventricle. In this case, thoracotomy is performed under anesthesia. After the multifunction catheter (2) is placed in the heart, the animal/subject is allowed to recover to a conscious state and return to a free-moving usual life.

After starting the device that has been set as above-mentioned, the conductance catheter (3) portion of the multifunction catheter (2) independently generates intracardiac electrocardiographic signals and conductance signals that are ventricular volume signals. These signals are detected by the corresponding signal detectors (7a, 7b, 7c and 8), amplified, and transmitted to the computing and processing units, etc. In addition, blood conductivity signals are generated and detected by the corresponding signal detector (6), amplified, and transmitted to the computing and processing unit.

Since there is a signal detector (8) for the intracardiac electrocardiographic signals, intracardiac electrocardiographic signals can be calculated in addition to the ventricular volume signals, which allows even more precise evaluation of the cardiac function.

By installing an electrode for measuring blood conductivity (4), blood conductivity can be determined using the electrode for measuring blood conductivity (4) and the electrode (32d₂ in the figure) in close proximity of the above electrode. For this reason, there is no need to perform blood sampling separately to measure blood conductivity for calculating ventricular volume. For instance, ventricular volume can be measured even when the experimental animals and the study operators are remotely located from each other.

By measuring conductance using two different frequencies, it is possible to measure parallel conductance derived from the myocardium and other sources, without performing special procedures such as saline infusion.

The practical measurement of parallel conductance will be described. First parallel conductance is measured using conventional methods such as the saline infusion method. Next, conductance is measured using three volume measurement segments. By measuring conductances using two different frequencies, the conductance. derived from the myocardium can be calculated. Next, the ratio (or coefficient) of the parallel conductance obtained from actual measurement to the parallel conductance obtained using different frequencies is calculated.

By calculating this coefficient in advance, parallel conductance can be determined by measuring the conductances using different frequencies, and then multiplying the difference in the above conductances by the coefficient.

Next, the automatic, continuous measuring device for a ventricle volume concerning the second embodiment of this invention will be explained with reference to the drawings. The automatic, continuous measuring device for a ventricle volume concerning the second embodiment of this invention differs from the automatic, continuous measuring device for a ventricle volume concerning the first embodiment by the following aspect. In the automatic, continuous measuring device for a ventricle volume concerning the first embodiment, blood conductivity is measured by the two-electrode measurement method using an electrode for measuring blood conductivity placed close to one of the multiple electrodes. In comparison, in the automatic, continuous measuring device for a ventricle volume concerning the second embodiment, blood conductivity is measured by the four-electrode measurement method.

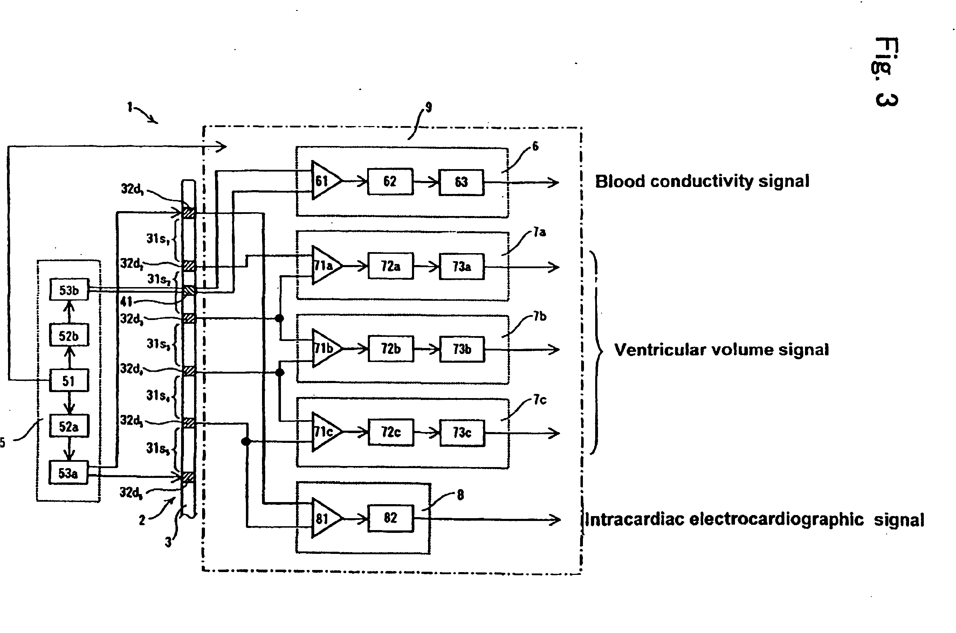

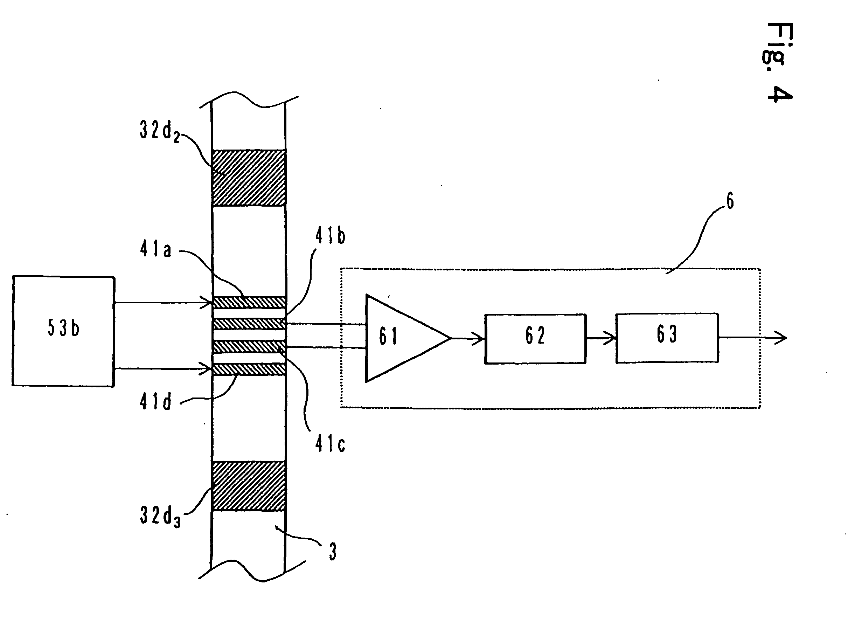

Figure 3 is the block diagram showing the outline of the automatic, continuous measuring device for a ventricle volume concerning the second embodiment of this invention. Figure 4 is the block diagram of the part around the electrode for measuring blood conductivity (41), enlarged and extracted from the block diagram of the automatic, continuous measuring device for a ventricle volume shown in Figure 3. Some components not essential for the explanation of Figure 4 have been omitted.

The electrode for measuring blood conductivity (41) is placed in one of the segments (31s₁ to 31s₅). In Figures 3 and 4, it is placed in segment (31s₂).

As shown in Figure 4, the electrode for measuring blood conductivity (41) is composed of 4 electrodes (41a to 41d) placed in fixed intervals. The electrode pair at the two ends (41 a and 41d) are current input electrodes, and the pair of electrodes (41 b and 41c) located in between the current input electrodes are voltage measurement electrodes.

When a current flows between the current input electrodes (41 a and 41 d), the blood between the current input electrodes (41a and 41 d) generate potential differences depending on the conductivity and distance. By measuring the voltage between the pair of voltage measurement electrodes (41b and 41 c) placed at equidistance between the current input electrodes (41a and 41d), the blood conductivity corresponding to the current can be measured.

The distances between the four electrodes (41a to 41d) are not specifically restricted. For example, the four electrodes (41 a to 41 d) can be placed at intervals of 0.1 mm.

The power supply unit (5) consists of a battery (51), a high frequency power circuit (52a) and a frequency modulating circuit (53a). The battery (51) supplies the necessary power to all parts of the signal detectors (7) and all parts of the automatic, continuous measuring device for a ventricle volume (1). In addition, the high frequency power circuit (52a) supplies an arbitrary high-frequency weak current that flows between the two electrodes (32d₁ and 32d₆) at the two ends of the conductance catheter (3). Furthermore, a frequency modulating circuit (53a) is installed so that the frequency of the weak current supplied to the conductance catheter (3) can be modulated arbitrarily.

Therefore, by measuring the intra-ventricular conductance using two different frequencies (for example, 20 KHz and 2 KHz); in other words, utilizing the difference in frequency characteristics of conductance between blood and the myocardium, it is possible to measure the parallel conductance derived from the myocardium and other sources. Therefore, parallel conductance, which is conventionally measured by hypertonic saline infusion and other methods, can be measured without special procedures.

In the measurement of parallel conductance using different frequencies, while the parallel conductance derived from the myocardium and other sources can be estimated by measuring ventricular conductance using two predetermined frequencies such as "20 KHz and 2 KHz", it is also possible to measure the ventricular conductance individually using three or more arbitrary frequencies (for example, 2 KHz, 20 KHz and 30 KHz) and then choose the two frequencies (for example, 2 KHz and 30 KHz) that give the greatest difference in conductance, and use them to measure parallel conductance. Using these methods, the parallel conductance, which is conventionally measured by hypertonic saline infusion and other methods, can be measured without special procedures and, moreover, with higher precision.

Furthermore, the power supply unit (5) contains a high frequency power circuit (52b) and a frequency modulating circuit (53b). Through the power supply from the high frequency power circuit (52b), an arbitrary high-frequency weak current flows between the current input electrodes (41 a and 41 d) at the two ends of the electrode for measuring blood conductivity (4) fitted in the conductance catheter (3). The frequency modulating circuit (53b) is installed so that the frequency of the weak current supplied by the current input electrodes (41a and 41d) can be modulated arbitrarily.

The signal detector (7) is the part that detects ventricular volume signals, intracardiac electrocardiographic signals and blood conductivity signals obtained from the multifunction catheter (2). As shown in Figure 3, the signal input side consists of multiple (three in the figure) signal detectors (7a, 7b and 7c) (hereinafter referred to collectively as 7) for detecting conductance signals that are the ventricular volume signals, one signal detector (8) for intracardiac electrocardiographic signals, and one signal detector (6) for blood conductivity signals. From these signal detectors (6, 7 and 8), the detected signals are output via four or more channels (five channels in the figure).

Among the above-mentioned signal detectors (6, 7 and 8), the detectors for ventricle volume signals are installed corresponding to the three conductance measuring segments (31s₂, 32s₃ and 32s₄) in the inner portion of the conductance catheter (3). They consist of differential amplifiers (71a, 71b and 71c) to measure signals from the electrodes (32d₂ and 32d₃) (32d₃ and 32d₄, or 32d₄ and 32d₅) at the two ends of each of the inner conductance measuring segments (31s₂) (31s₃ or 31s₄), band pass filters (72a, 72b and 72c), and wave detectors (73a, 73b and 73c). The band pass filters (72a, 72b and 72c) eliminate contamination by other signals such as intracardiac electrocardiographic signals. The wave detectors (73a, 73b and 73c) cut off the high frequency components from the output of the band pass filters (72a, 72b and 72c) and extract the low frequency components.

The signal detector (8) for intracardiac electrocardiographic signals consists of a differential amplifier (81) connected to the two electrodes (32d₁ and 32d₅) at the two ends of the conductance catheter (3), and a band pass filter (82).

The signal detector (6) for blood conductivity signals consists of a differential amplifier (61) connected to the two central electrodes for measuring blood conductivity (41 b and 41 c), and a band pass filter (62).

Then, various signals output from the signal detectors (6, 7 and 8) and the information concerning the current supplied to the current input electrodes (41a and 41d) are transmitted to the computing and processing unit (not shown in the figure) and undergo specified processing.

Various signals output from the signal detectors (6,7 and 8) and the information concerning the current supplied to the current input electrodes (41 a and 41 d) are transmitted to the computing and processing unit (not shown in the figure) and undergo specified processing.

When the conductance catheter (3) is inserted into the heart for a long period of time, the blood components may adhere to the surrounding of the conductance catheter (3). When blood conductivity is measured by two-electrode measurement, the blood components attached to the surrounding of the conductance catheter (3) change the voltage, and accurate measurement of blood conductivity may not be possible.

On the other hand, when blood conductivity is measured by four-electrode measurement, even if blood components are adhered to the surrounding of the conductance catheter, the effect of this adhered blood components on blood conductivity is relatively small, and more accurate measurement of blood conductivity compared to the two-electrode measurement method is possible.

Next, the operations of the automatic, continuous measuring device for a ventricle volume concerning the second embodiment of this invention will be explained.

For measurement, the multifunction catheter (2) is inserted into the ventricle. In this case, thoracotomy is performed under anesthesia. After the multifunction catheter (2) is placed in the heart, the animal/subject is allowed to recover to a conscious state and return to a free-moving usual life.

After starting the device that has been set as above-mentioned, the conductance catheter (3) portion of the multifunction catheter (2) generates intracardiac electrocardiographic signals and conductance signals that are ventricular volume signals. These signals are detected by the corresponding signal detectors (7a, 7b, 7c and 8), amplified, and transmitted to the computing and processing units, etc. In addition, blood conductivity signals are generated and detected by the corresponding signal detector (6), amplified, and transmitted to the computing and processing unit.

In the automatic, continuous measuring device for a ventricle volume concerning the second embodiment, blood conductivity can be measured by the four-electrode measurement method using electrodes for measuring blood conductivity (41) composed of a pair of current input electrodes (41 a and 41 d) and a pair of voltage measurement electrodes (41b and 41c). For this reason, there is no need to perform blood sampling separately to measure blood conductivity for the calculation of ventricular volume. For instance, ventricular volume can be measured even when the experimental animals and the study operators are physically remote from each other. Furthermore, this method has less measurement error compared to the two-electrode measurement method for measuring blood conductivity, and more precise measurement of ventricular volume is thus possible.

Components other than those described in detail above are the same as the above-mentioned automatic, continuous measuring device for a ventricle volume (1) concerning the first embodiment. Their explanations are omitted.

The automatic, continuous measuring device for a ventricle volume (1) concerning the first and second embodiments is well suited for use in small animals such as rats and mice; as well as in large animals such as dogs, cats, cows, sheep, horses and humans.

As described in detail above, for the invention concerning claim 1, placement of an electrode for measuring blood conductivity close to one of the electrodes installed in the conductance catheter obviates the need to collect blood and measure blood conductivity separately. For that reason, for instance, ventricular volume can be measured continuously even when the experimental animals and the operators are physically remote from each other.

For the invention concerning claim 2, since blood conductivity can be measured using the four-electrode measurement method, even though the multifunction catheter has been inserted in the ventricle for a long period of time, the error of blood conductivity measurement due to adhesion of blood components on the multifunction catheter can be kept to the minimum, permitting accurate ventricular volume measurement.

For the invention concerning claim 3, since the device is able to supply a weak current of two frequencies to the electrodes at the two ends of the multifunction catheter, conductances for two different frequencies can be measured. Therefore, by calculating the difference of the above-mentioned conductances, the conductance derived from the myocardium and other sources can be determined. Using the previously determined coefficient, the parallel conductance can be calculated. This method permits automatic measurement of ventricular volume continuously without the need to measure parallel conductance of the experimental animal by conventional methods such as saline infusion.

For the invention concerning claim 4, by measuring conductance at three or more frequencies and then using the two frequencies that show the greatest difference in measured conductance to measure parallel conductance, the error of ventricular volume measurement can be reduced.

### <Test Examples>

This invention will be explained in detail based on tests as shown below.

### Test 1: Inter-electrode distance and effective conducting volume measurement

The relationship between inter-electrode distance and effective conducting volume measurement was examined when a constant current is passed between two electrodes on a catheter placed in homogenous isotropic conductance.

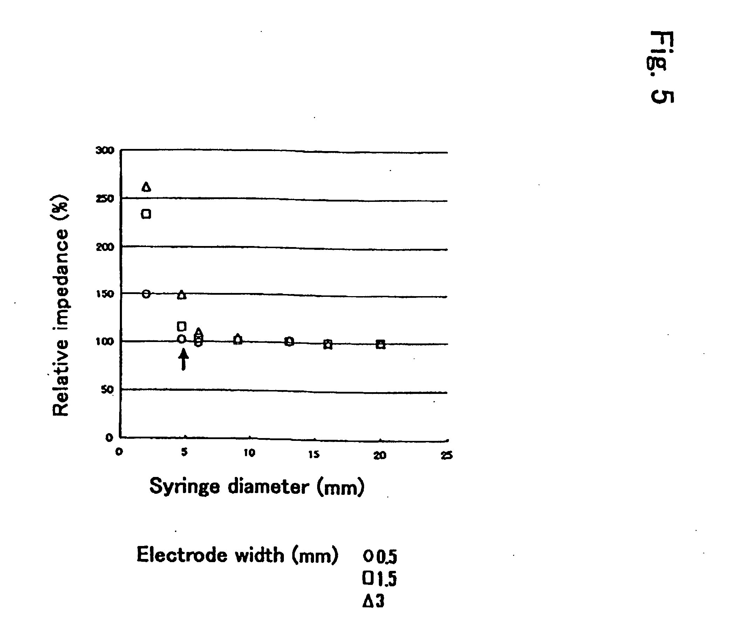

First, three types of conductance catheters with platinum electrodes (width 0.5 mm) set at three inter-electrode distances (3.0 mm, 1.5 mm and 0.5 mm) were placed in the center of syringes filled with dilute physiological saline having diameters varying step-wise from 2 to 20 mm. The conductivity of the dilute physiological saline was set to a value resembling that of blood (6.6 ms/cm). Next, a constant current (20 KHz, 30 µA) is passed between two electrodes and the voltage between electrodes was measured. From this voltage, the magnitude of impedance between two electrodes was determined. The results of impedance for each syringe diameter were expressed as relative values (%) using the impedance of the 20 mm syringe as standard. The results are shown in Figure 5.

As shown in the results of Figure 5, the relative impedance decreased with increase in syringe diameter for all the electrode types, and the relative impedance reached 100% at syringe diameters of 10 mm and greater for all the inter-electrode distances. In addition, with shorter inter-electrode distance, impedance decrease was observed with smaller syringe diameters. In the case of an inter-electrode distance of 0.5 mm, the relative impedance was 100% at a syringe diameter of 5 mm.

The above results indicate that when the inter-electrode distance is within 3 mm, the effective conductivity volume can be focused within diameter of 10 mm vertical to the catheter, and when the distance between two electrodes is 0.5 mm, it can be focused within a diameter of 5 mm.

### Test 2: Blood conductivity measurement

A conductance catheter was placed into the left ventricle of a Japanese white rabbit. The conductance catheter had an electrode for measuring blood conductivity placed 0.5 mm from one (third electrode from the catheter tip) of 7 electrodes that formed the conductance catheter. A high-frequency current of 20 KHz and 30 pA was passed between the two ends of the conductance catheter and the blood conductivity (σv) was measured. On the other hand, blood was collected from the same Japanese white rabbit and the blood conductivity (σc) was measured using a special cuvette.

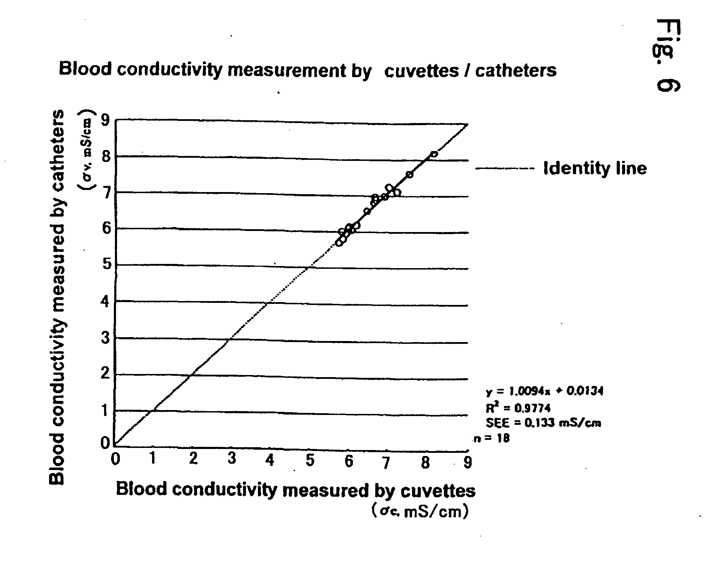

Measurements were made in 18 rabbits under the same conditions. The results are shown in Figure 6. The X axis in Figure 6 is the blood conductivity measured by the cuvette (σc) and the Y axis is the blood conductivity measured by the catheter (σv).

As shown in Figure 6, a strong correlation was observed between σc and σv.

### Test 3: Parallel conductance measurement

In the Japanese white rabbits (18 in total) implanted with the conductance catheter in the left ventricle, a high-frequency current of 20 KHz and 2 KHz (30 µA) was passed between the two ends of the conductance catheter and the differences of the mean blood conductivity signals for the two frequencies (ΔG 2 KHz-20 KHz) were determined.

Next, nine Japanese white rabbits were randomly selected and parallel conductance at 20 KHz (GpSAL) was measured by the saline infusion method. GpSAL and ΔG 2KHz-20KHz showed a correlation represented by the equation: GpSAL= 9.1021×ΔG 2KHz-20KHz (R2=0.87). Next, the GpSAL : ΔG 2KHz-20KHz ratio was calculated for each Japanese white rabbit, and the mean value was 9.158 (±0.558).

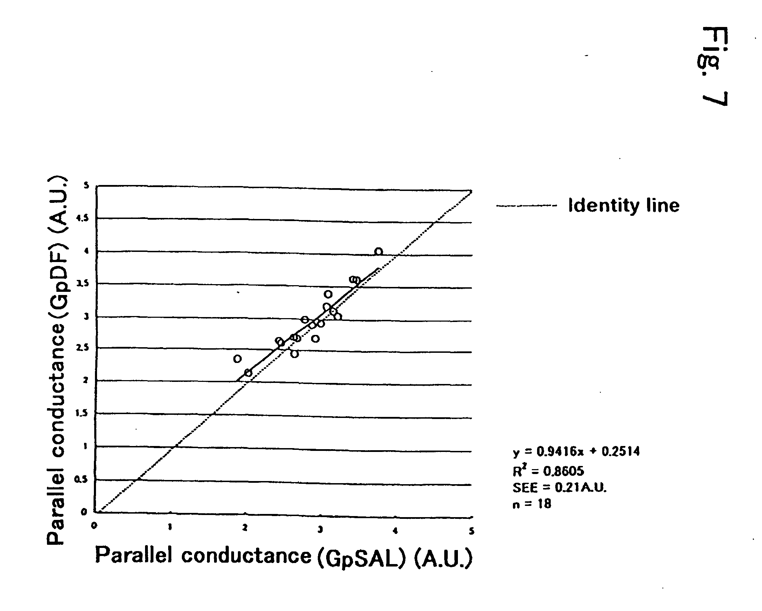

Using the value 9.158 (±0.558) as the empirical coefficient, the parallel conductance calculated from the ΔG 2KHz-20KHz values (GpDF) showed a good correlation with the corresponding GpSAL values. This coefficient was applied to the remaining 9 rabbits. For all 18 animals, a correlation between GpDF and GpSAL was observed, as shown in Figure 7. These results confirmed that this empirical coefficient can be applied universally in this animal species.

Next, the highest and lowest conductance signals (36 in total) obtained from the 18 rabbits were used in the following analysis. The correlation between the diastolic and systolic ventricular volumes calibrated using σc and GpSAL according to the conventional method (Vc) and the diastolic and systolic ventricular volumes calculated from GpDF (Ve) was analyzed. The results are shown in Figure 8.

As shown in Figure 8, a good correlation was observed between Vc determined by the conventional method and Ve measured using the device concerning this invention.

Ve and Vc showed good agreement even by the Bland-Altman analysis.

The above results indicate that the device concerning this invention is able to measure ventricular volume continuously without the calibration procedures used in the conventional method.

### Industrial Applicability

The automatic, continuous measuring device for a ventricle volume concerning this invention permits measurement of blood conductivity and parallel conductance, which are regarded as essential for calculating ventricular volume from the conductance measured by a conductance catheter, by using the conductance catheter inserted into the ventricle without special procedures such as blood sampling and saline infusion. It allows automatic and, moreover, continuous measurement of the ventricular volume even when the operator is not physically close to the animal implanted with the conductance catheter.

## Claims

1. An automatic, continuous measuring device for a ventricle volume (1) **characterized by** the following: furnished with a multifunction catheter (2) to be introduced into the ventricle, and a power supply unit (5) to supply the necessary current to the above-mentioned multifunction catheter (2);
The above-mentioned multifunction catheter (2) consists of two parts - a conductance catheter (3) made up of multiple segments interposed with electrodes at fixed intervals such that an arbitrary high-frequency weak current is passed between the two end electrodes while the voltages are measured in the other segments except the two end segments, and an electrode for measuring blood conductivity (4) placed at one of the above-mentioned multiple segments.

2. An automatic, continuous measuring device for a ventricle volume (1) **characterized by** the following: furnished with a multifunction catheter (2) to be introduced into the ventricle, and a power supply unit (5) to supply the necessary current to the above-mentioned multifunction catheter (2);
The above-mentioned multifunction catheter (2) consists of two parts - a conductance catheter (3) made up of at least three segments interposed with at least four electrodes such that an arbitrary high-frequency weak current is passed between the two end electrodes while the voltages are measured in all segments except the two end segments, and four electrodes (41) for measuring blood conductivity placed in one of the above-mentioned multiple segments;
Of the above-mentioned four electrodes (41) for measuring blood conductivity, the electrodes at two ends are designated current input electrodes (41 a and 41d) and the inner pair of electrodes is designated voltage measurement electrodes (41 b and 41 c).

3. An automatic, continuous measuring device for a ventricle volume (1) described in claim 1 or claim 2 **characterized by** the following: the above-mentioned power supply unit (5) is configurated so as to be able to supply a weak current at two frequencies between the two electrodes at the two ends of the above-mentioned multifunction catheter (2).

4. An automatic, continuous measuring device for a ventricle volume (1) described in claim 1 or claim 2 **characterized by** the following: the above-mentioned power supply unit (5) is configurated so as to be able to supply a weak current at three or more frequencies between the two electrodes at the two ends of the above-mentioned multifunction catheter (2);
Individual ventricular conductances are measured with three or more arbitrary frequencies supplied to the electrodes at the two ends of the above-mentioned multifunction catheter (2), and the two frequencies that provide the greatest difference in conductance are used to determine parallel conductance.
